# EUROPEAN PATENT SPECIFICATION

(11) **EP 2 600 813 B1**
(45) Date of publication and mention of the grant of the patent: **20.01.2021**
(21) Application number: 11815199.2
(22) Date of filing: 02.08.2011
(51) Int. Cl.: A61F 9/007, B25J 13/08, B25J 19/02, A61B 34/00

(54) **SURGICAL SYSTEM USING COOPERATIV MANUAL/ROBOT-CONTROL AND AUDIO FEEDBACK**
CHIRURGISCHES SYSTEM MIT KOOPERATIVER MANUELLER/ROBOTER-STEUERUNG UND EIN AUDIOFEEDBACK
SYSTÈME CHIRURGICAL AVEC CONTRÔLE COOPÉRATIF MANUEL/ROBOT ET RETOUR AUDIO

(30) Priority: 02.08.2010 US 370029 P
(43) Date of publication of application: 12.06.2013
(73) Proprietor: The Johns Hopkins University, Baltimore, MD 21218 (US)
(72) Inventor: TAYLOR, Russell, H., Severna Park MD 21246 (US); BALICKI, Marcin, Arkadiusz, Baltimore MD 21211 (US); HANDA, James, Tahara, Baltimore MD 21212 (US); GEHLBACH, Peter, Louis, Hunt Valley, MD 21030 (US); IORDACHITA, Iulian, Towson MD 21204 (US); UNERI, Ali, Baltimore, MD 21210 (US)
(74) Representative: Murgitroyd & Company
(86) International application number: PCT/US2011/046276
(87) International publication number: WO 2012/018821

(56) References cited:
- WO-A1-2009/034962
- WO-A1-2009/124287
- WO-A2-2009/140688
- JP-A- 2003 211 377
- US-A1- 2002 120 188
- US-A1- 2009 076 476

## Description

### FIELD OF THE INVENTION

The present invention pertains to a system for cooperative control for surgical tools. More particularly, the present invention pertains to a system for presenting force sensor information using cooperative robot control and audio feedback.

### BACKGROUND OF THE INVENTION

Retinal microsurgery is one of the most challenging set of surgical tasks due to human sensory-motor limitations, the need for sophisticated and miniature instrumentation, and the inherent difficulty of performing micron scale motor tasks in a small and fragile environment. In retinal surgery, surgeons are required to perform micron scale maneuvers while safely applying forces to the retinal tissue that are below sensory perception. Surgical performance is further challenged by imprecise instruments, physiological hand tremor, poor visualization, lack of accessibility to some structures, patient movement, and fatigue from prolonged operations. The surgical instruments in retinal surgery are characterized by long, thin shafts (typically 0.5 mm to 0.7 mm in diameter) that are inserted through the sclera (the visible white wall of the eye). The forces exerted by these tools are often far below human sensory thresholds.

The surgeon therefore must rely on visual cues to avoid exerting excessive forces on the retina. These visual cues are a direct result of the forces applied to the tissue, and a trained surgeon reacts to them by retracting the tool and re-grasping the tissue in search of an alternate approach. This interrupts the peeling process, and requires the surgeon to carefully re-approach the target. Sensing the imperceptible micro-force cues and preemptively reacting using robotic manipulators has the potential to allow for a continuous peel, increasing task completion time and minimizing the risk of complications. All of these factors contribute to surgical errors and complications that may lead to vision loss.

An example procedure is the peeling of the epiretinal membrane, where a thin membrane is carefully delaminated off the surface of the retina using delicate (20-25 Ga) surgical instruments. The forces exerted on retinal tissue are often far below human sensory thresholds. In current practice, surgeons have only visual cues to rely on to avoid exerting excessive forces, which have been observed to lead to retinal damage and hemorrhage with associated risk of vision loss.

Although robotic assistants such as the DAVINCI™ surgical robotic system have been widely deployed for laparoscopic surgery, systems targeted at microsurgery are still at the research stage. Microsurgical systems include teleoperation systems, freehand active tremor-cancellation systems, and cooperatively controlled hand-over-hand systems, such as the Johns Hopkins "Steady Hand" robots. In steady-hand control, the surgeon and robot both hold the surgical tool; the robot senses forces exerted by the surgeon on the tool handle, and moves to comply, filtering out any tremor. For retinal microsurgery, the tools typically pivot at the sclera insertion point, unless the surgeon wants to move the eyeball. This pivot point may either be enforced by a mechanically constrained remote center-of-motion or software. Interactions between the tool shaft and sclera complicate both the control of the robot and measurement of tool-to-retina forces.

To measure the tool-to-retina forces, an extremely sensitive (0.25 mN resolution) force sensor has been used, which is mounted on the tool shaft, distal to the sclera insertion point. The force sensor allows for measurement of the tool tissue forces while diminishing interference from tool-sclera forces. In addition, endpoint micro-force sensors have been used in surgical applications, where a force scaling cooperative control method generates robot response based on the scaled difference between tool-tissue and tool hand forces.

In addition, a first-generation steady-hand robot has been specifically designed for vitreoretinal surgery. While this steady-hand robot was successfully used in ex-vivo robot assisted vessel cannulation experiments, it was found to be ergonomically limiting. For example, the first generation steady-hand robot had only a ±30% tool rotation limit. To further expand the tool rotation range, a second generation steady-hand robot has been developed which has increased this range to ±60%. The second generation steady-hand robot utilizes a parallel six-bar mechanism that mechanically provides isocentric motion, without introducing large concurrent joint velocities in the Cartesian stages, which occurred with the first generation steady-hand robots.

The second generation steady-hand robot incorporates both a significantly improved manipulator and an integrated microforce sensing tool, which provides for improved vitreoretinal surgery. However, because of the sensitivity of vitreoretinal surgery, there is still a need in the art for improved control of the tool, to avoid unnecessary complications. For example, complications in vitreoretinal surgery may result from excess and/or incorrect application of forces to ocular tissue. Current practice requires the surgeon to keep operative forces low and safe through slow and steady maneuvering. The surgeon must also rely solely on visual feedback that complicates the problem, as it takes time to detect, assess and then react to the faint cues; a task especially difficult for novice surgeons.

Accordingly, there is a need in the art for an improved control method for surgical tools used in vitreoretinal surgery and the like.

WO 2009/124287 A1 discloses a system for inserting a steerable array into an anatomical structure of the body. The system includes an insertion module for holding a proximal end of the steerable array and a force sensor configured to detect force on the steerable array and to produce force information. A stop signal mechanism may be implemented in order to avoid damaging contact between the steerable array and the anatomical structure. In some instances, this mechanism will relay information to the surgeon by generating a visual, audio and/or vibration cue.

US 2009/076476 A1 discloses a medical instrument system including a controller and a guide instrument coupled to an instrument driver, the instrument driver configured to manipulate a distal end portion of the guide instrument in response to control signals generated by the controller. A force sensor is associated with the guide instrument or with a working instrument carried by the guide instrument, and generates force signals responsive to a force applied to a respective distal end portion of the guide instrument or working instrument.

US 2002/120188 A1 discloses an apparatus for sensing at an anatomic body site and mapping or transforming the sensor signal into various forms of virtual image and feedback signals, having particular application in assisting surgeons and other operators during a medical procedure.

### SUMMARY

According to a first aspect of the present invention, a system for cooperative control of a surgical tool comprises a tool holder for receiving a surgical tool adapted to be held by a robot and a surgeon, a sensor for detecting a force based on operator input and/or tool tip forces, a controller for limiting robot velocity based upon the force detected between the surgical tool and the tissue so as to provide a haptic feedback, a selector for automatically selecting one level of a multi-level audio feedback based upon the detected force applied, the audio feedback representing the relative intensity of the force applied, and an audio device for providing the audio feedback together with the haptic feedback.

According to a second aspect of the present invention, a system for cooperative control of a surgical tool comprises a tool holder for receiving a surgical tool adapted to be held by a robot and a surgeon, a sensor for detecting a distance between a surgical tool and a target area of interest, a selector for automatically selecting an audio feedback based upon the detected distance, the audio feedback representing range sensing information regarding how far the surgical tool is from the target area of interest, and an audio device for providing the audio feedback.

Further, a method for cooperative control of a surgical tool comprises receiving a surgical tool adapted to be held by a robot and a surgeon, detecting a force at an interface between the surgical tool and tissue, limiting robot velocity based upon the force detected between the surgical tool and the tissue so as to provide a haptic feedback, automatically selecting an audio feedback based upon the detected force, the audio feedback representing the relative intensity of the force applied, and providing the selected audio feedback together with the haptic feedback.

### BRIEF DESCRIPTION OF THE DRAWINGS

The accompanying drawings provide visual representations which will be used to more fully describe the representative embodiments disclosed herein and can be used by those skilled in the art to better understand them and their inherent advantages. In these drawings, like reference numerals identify corresponding elements and:
FIG. 1 illustrates a schematic of an exemplary system according to the features of the present invention.
FIG. 2 illustrates a schematic of an exemplary system according to the features of the present invention.
FIG. 3 illustrates an exploded view of an exemplary surgical tool according to the features of the present invention.
FIG. 4 illustrates a graphical representation of the audio feedback with respect to force according to the features of the present invention.
FIG. 5 illustrates a graphical representation of the peeling sample repeatability tests according to features of the present invention.
FIGS 6 A-D are plots of representative trials of various control modes showing tip forces, with and without audio feedback according to features of the present invention.

### DETAILED DESCRIPTION OF THE PREFERRED EMBODIMENTS

The presently disclosed subject matter now will be described more fully hereinafter with reference to the accompanying Drawings, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Drawings. Therefore, it is to be understood that the presently disclosed subject matter is not to be limited to the specific embodiments disclosed and that modifications and other embodiments are intended to be included within the scope of the appended claims.

The present invention pertains to a system and method for cooperative control of a surgical tool. An exemplary embodiment of the invention provides for use of the system and method in cooperatively controlled hand-over-hand systems, such as the robotic assisted surgical system described in "Development and Application of a New Steady-Hand Manipulator for Retinal Surgery", Mitchell et al., IEEE ICRA, pp. 623-629 (2007), in "Micro-force Sensing in Robot Assisted Membrane Peeling for Vitreoretinal Surgery", M. Balicki, A. Uneri, I. Iordachita, J. Handa, P. Gehlbach, and R. H. Taylor, Medical Image Computing and Computer-Assisted Intervention (MICCAI), Beijing, September, 2010, pp. 303-310, and in "New Steady-Hand Eye Robot with Microforce Sensing for Vitreoretinal Surgery Research", A. Uneri, M. Balicki, James Handa, Peter Gehlbach, R. Taylor, and I. Iordachita, International Conference on Biomedical Robotics and Biomechatronics (BIOROB), Tokyo, September 26-29, 2010, pp. 814-819. In steady-hand control, the surgeon and robot both hold the surgical tool. The robot senses forces exerted by the surgeon on the tool handle, and moves to comply, filtering out any tremor. While a specific cooperative control system is described in connection with the above publication, it should be understood that the system of the present invention may also be applicable to other cooperatively controlled systems, as well as freehand surgery.

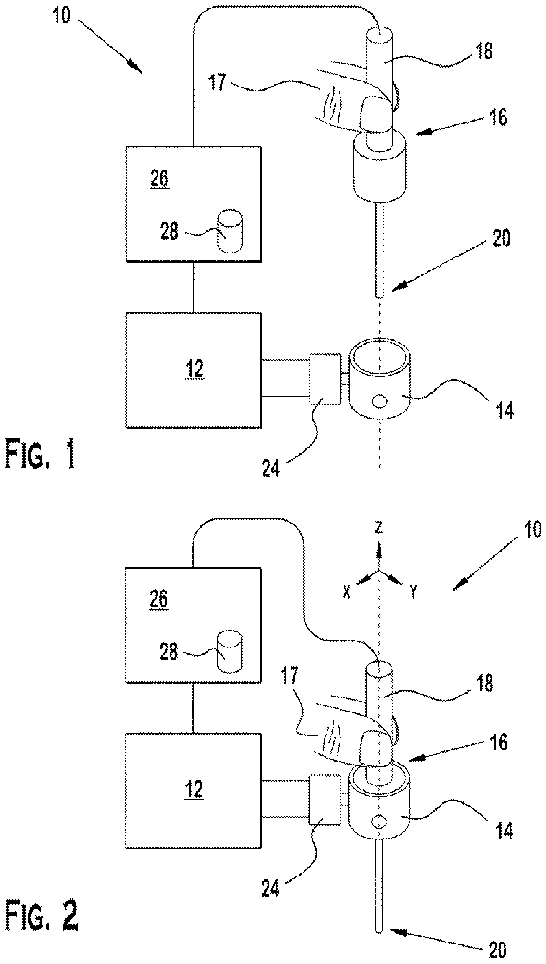

With reference to FIGS. 1 and 2, a first illustrative embodiment of a robotic-assisted surgical system to be used in connection with the present invention is shown. The system 10 may be used, for example, in micro-surgery of organs, for example, hollow organs, such as the human eye, but other applications are possible.

As shown in FIGS. 1 and 2, the system 10 includes a tool holder 14 for receiving a surgical tool 16 to be held both a robot 12 and a surgeon 17. The tool holder 14 facilitates the attachment of a variety of surgical tools required during microsurgical procedures, including but not limited to, forceps, needle holder, and scissors. Preferably, the surgeon 17 holds the surgical tool 16 at a tool handle 18, and cooperatively directs the surgical tool 16 with the robot 12 to perform surgery of a region of interest with a tool tip 20. In addition, a force/torque sensor 24 may be mounted at the tool holder 16, which senses forces exerted by the surgeon on the tool, for use as command inputs to the robot.

Preferably, a custom mechanical RCM is provided, which improves the stiffness and precision of the robot stages. The RCM mechanism improves the general stability of the system by reducing range of motion and velocities in the Cartesian stages when operating in virtual RCM mode, which constrains the tool axis to always intersect the sclerotomy opening on the eye.

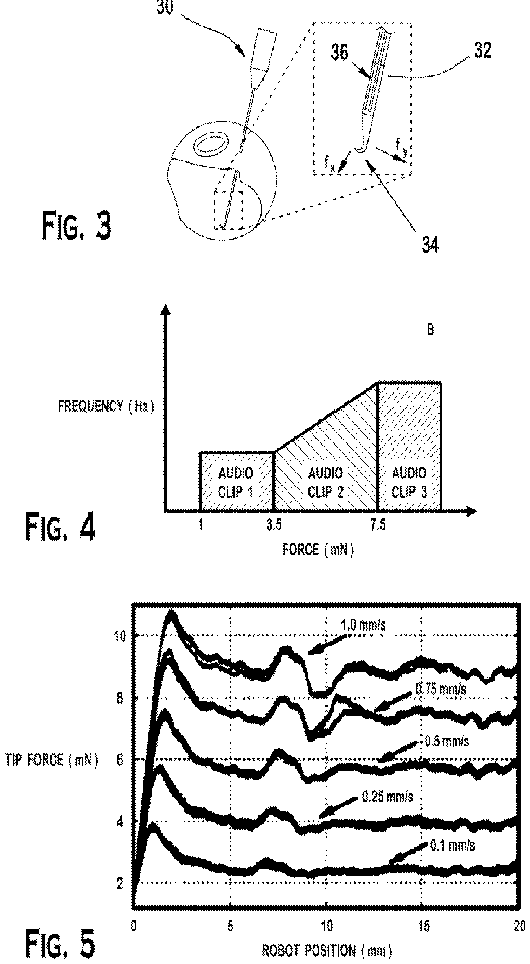

With reference to FIG. 3, an exemplary surgical tool 30 to be used in connection with the system of the present invention is illustrated. In particular, surgical tool 30 may be specifically designed for use in a cooperative manipulation, such as a system describe above, but may be used in a tele-operative robot as an end effector of a surgical robot or for freehand manipulation. In addition, the surgical tool 30 may be specifically designed for operation on the human eye E.

With continued reference to FIG. 3, the surgical tool 30 includes a tool shaft 32 with a hooked end 34. The surgical tool 30 preferably is manufactured with integrated fiber Bragg grating (FGB) sensors. FBGs are robust optical sensors capable of detecting changes in stain, without interference from electrostatic, electromagnetic or radio frequency sources. Preferably, a number of optical fibers 36 are placed along the tool shaft 32, which allows measuring of the bending of the tool and for calculation of the force in the transverse plane (along _{Fx} and _{Fy}) with a sensitivity of 0.25 mN. Accordingly, a sensitive measurement of the forces between the tool and tip can be obtained.

For vitreoretinal microsurgical applications, a force sensor should be chosen that allows for sub-mN accuracy, requiring the sensing of forces that are routinely below 7.5mN. As such a very small instrument size is necessary to be inserted through a 25 Ga sclerotomy opening and the force sensor is designed to obtain measurements at the instrument's tip, below the sclera.

With reference back to FIGS. 1 and 2, the system 10 includes a processor 26 and a memory device 28. The memory device 28 may include one or more computer readable storage media, as well as machine readable instructions for performing cooperative control of the robot. According to features of the claimed invention, depending upon the forces detected which are sent to the processor 26 (operator input and/or tool tip forces), robot velocity is limited by a controller so as to provide a haptic feedback. In addition, the program includes instructions for automatically selecting one level of a multi-level audio feedback based upon the detected force applied. The audio feedback represents the relative intensity of the force applied. An audio device provides for the audio feedback together with the haptic feedback. Preferably, the audio device is integral with the processor 26, but may also be a separate device.

With reference to FIG. 4, an exemplary embodiment of the multi-level audio feedback is graphically represented. In particular, a useful range of audio feedback was developed specifically for vitreoretinal surgery. In particular, auditory feedback that modulates the playback tempo of audio "beeps" in three force level zones were chosen to present force operating ranges that are relevant in typical vitreoretinal operations. The audio feedback may be selected based upon whether the applied force falls within a predetermined range. According to the preferred embodiment, the audio may be silent until 1 mN or greater force is measured. A constant slow beeping was chosen from the range of 1 mN until about 3.5mN, which is designated to be the "safe" operating zone. A "cautious" zone was designated as 3.5- 7.5 mN, and had a proportionally increasing tempo followed by a "danger zone" that generates a constant high tempo beeping for any force over 7.5 mN. In addition, the high tempo beeping preferably increases proportionally to the force applied. to further indicate to the surgeon that excessive forces are being applied.

As discussed above, there are different cooperative control methodologies that modulate the behavior of the robot based on operative input and/or tool tip forces, and can be used in connection with audio feedback as described in accordance the present invention. The control method parameters considered handle input force range (0-5N), and peeling task forces and velocities. Audio sensory substitution serves as a surrogate or complementary form of feedback and provides high resolution real-time tool tip force information. However, it should be understood that different types of control methods may be used in connection with the audio feedback, in accordance with features of the present invention. In addition, it should be understood that other types of audio feedback are included in the present invention, and are not limited to beeps.

One example of a cooperative control method is a proportional velocity control (PV) paradigm as described in "Preliminary Experiments in Cooperative Human/Robert Force Control for Robot Assisted Microsurgical Manipulation", Kumar et al., IEEE ICRA, 1:610-617 (2000). In particular, the velocity of the tool (V) is proportional to the user's input forces at the handle (Fₕ). For vitreoretinal surgery, a gain of α = 1 was used, which translates handle input force of 1 N to 1 mm/s tool velocity.

Another cooperative control method is called linear force scaling control (FS), which maps, or amplifies, the human-imperceptible forces sensed by the tool tip (Fₜ) to handle interaction forces by modulating robot velocity. Prior applications used γ = 25 and γ = 62.5 scale factors (which are low for the range of operating parameters in vitreoretinal peeling), as described in "Evaluation of a Cooperative Manipulation Microsurgical Assistant Robot Applied to Stapedotomy", Berkelman et al., LNCS ISSU 2208: 1426-1429 (2001) and "Preliminary Experiments in Cooperative Human/Robert Force Control for Robot Assisted Microsurgical Manipulation", Kumar et al., IEEE ICRA, 1:610-617 (2000). Scaling factor of γ = 500 can be used to map the 0 - 10mN manipulation forces at the tool tip to input forces of 0 - 5 N at the handle.

Another cooperative control method that can be used in connection with the present invention is proportional velocity control with limits (VL), which increases maneuverability when low tip forces are present. The method uses PV control, but with an additional velocity constraint that is inversely proportional to the tip force. With such scaling, the robot response becomes very sluggish with higher tool tip forces, effectively dampening manipulation velocities. For vitreoretinal surgery, the constraint parameters were chosen empirically to be m=-180 and b= 0.9. To avoid zero crossing instability, forces lower than f₁ = 1 mN in magnitude do not limit the velocity. Likewise, to provide some control to the operator when tip forces are above a high threshold (f₂ = 7.5 mN), a velocity limit (v₂ = 0.1) is enforced.

The present invention is also useful for freehand surgery. In current practice, surgeons indirectly assess the relative stress applied to tissue via visual interpretation of changing light reflections from deforming tissue. This type of "visual sensory substitution" requires significant experience and concentration, common only to expert surgeons. To provide more clear and objective feedback, forces may be measured directly and conveyed to the surgeon in real time with auditory representation, according to features of the present invention.

The present invention may also be used in connection with detecting how far the surgical tool is from the target area of interest. In particular, a sensor may be provided for detecting the distance between the surgical tool and the target area of interest. An audio feedback is selected based upon the detected distance. Preferably, the sensor is an OCT range sensor, but may include any other type of distance sensor.

### EXAMPLE

The following Example has been included to provide guidance to one of ordinary skill in the art for practicing representative embodiments of the presently disclosed subject matter. In light of the present disclosure and the general level of skill in the art, those of skill can appreciate that the following Example is intended to be exemplary only and that numerous changes, modifications, and alterations can be employed without departing from the scope of the presently disclosed subject matter. The following Example is offered by way of illustration and not by way of limitation.

A tool with intergrated fiber Bragg grating (FBG) sensors was manufactured with three optical fibers along the tool shaft. The tool was mounted in the robot tool holder in a calibrated orientation relative to the robot. The sensor data was collected and processed at 2 kHz and transmitted over TCP/IP. To simulate the peeling of retinal tissue, a phantom model was generated. Sticky tabs from 19 mm Clear Bandages (RiteAid brand) were found to be a suitable and repeatable phantom for delaminating. The tab was sliced to produce 2 mm wide strips that can be peeled multiple times from its backing, with predictable behavior showing increase of peeling force with increased peeling velocity. The plastic peeling layer was very flexible but strong enough to withstand breaking pressures at the hook attachment site. 20 mm of tool travel was needed to complete a peel. Fig. 5 shows the forces observed at various velocities.

The effectiveness of the control methods described above were compared with regard to decreasing mean and maximum peeling forces while minimizing the time taken to complete the task. A single subject was tested in this example, which was configured in the following ways. The phantom was adhered to a stable platform with double-stick tape and the robot was positioned so the hook is ∼1.5 mm above the peeling surface. The orientation of the handle was perpendicular to the peeling direction and comfortable to the operator. To eliminate force cues from tool bending, the visibility of the tool shaft was obstructed with the exception of the tool tip. The test subject was trained extensively (∼3 hours) prior to the trials. Five minute breaks were allowed between trials. The operator was directed to peel the membrane steadily and as slow as possible without stopping. To simplifying the experiments, the robot motion was limited to Cartesian translations only; experiments showed no noticeable difference between trials with and without rotational DOFs. No visual magnification was provided to the operator. For all trials, the same sample was used and, for consistency, the behavior of the sample before and after the experiment was tested. For comparison, freehand peeling tests where the operator peeled the sample without robot assistance were included. Five trials of each method were performed with audio feedback, and five without.

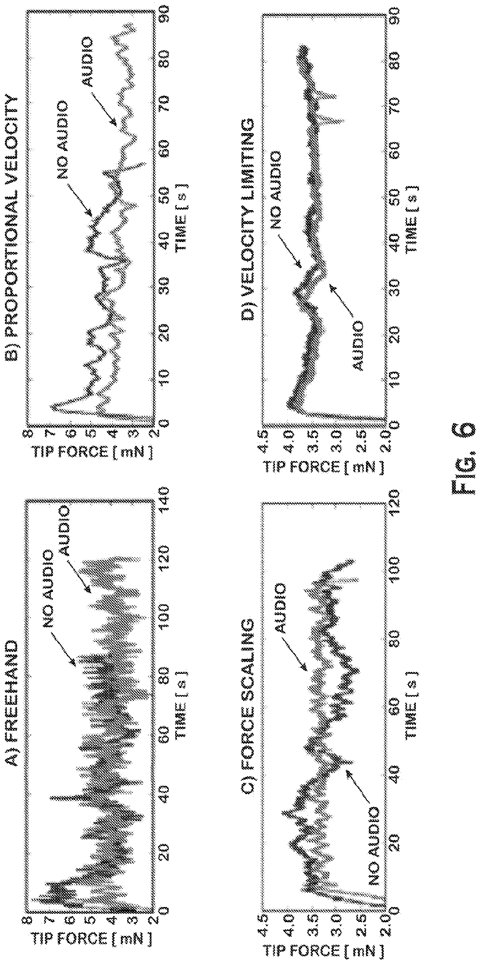

In every method tested, audio feedback decreased the maximum tip forces, as well as tip force variability. It significantly increased the task completion time for freehand and proportional velocity control trials while the time decreased slightly for the others. The operator was naturally inclined to "hover" around the discrete audio transition point corresponding to 3.5 mN, which was observed in all cases except freehand. This was particularly prominent in force scaling, where the operator appears to rely on audio cues over haptic feedback (see Fig. 5C, time 60-80 s). In velocity limiting trials, audio reduced mean input handle forces by 50% without compromising performance. This indicates that the user consciously attempted to use audio feedback to reduce the forces applied to the sample.
***Freehand*** (Fig. 6A) trials showed considerable high force variation due to physiological hand tremor. The mean force applied was around 5 mN, with maximum near 8 mN. Audio feedback helped to reduce large forces but significantly increased task completion time.
***Proportional Velocity*** (Fig. 6B) control performance benefited from the stability of robot assistance and resulted in a smoother force application, while the range of forces was comparable to freehand tests. Likewise, audio feedback caused a decrease in large forces but increased time to complete the task.
***Force Scaling*** (Fig. 6C) control yielded the best overall performance in terms of mean forces with and without audio. Although, the average time to completion was the longest, except for freehand with audio.
***Velocity Limiting*** (Fig. 6D) control resulted in a very smooth response except for the section that required higher absolute peeling forces at the limited velocity. This had an effect of contouring "along" a virtual constraint. Due to matching thresholds, audio had very little effect on the performance.

Accordingly to experimental data above, the present invention provides a system capable of measuring and reacting to forces under 7.5 mN, a common range in microsurgery. In addition, the force scaling together with audio feedback provides the most intuitive response and force-reducing performance in a simulated membrane peeling task, where the goal is to apply low and steady forces to generate a controlled delamination.

Although the present invention has been described in connection with preferred embodiments thereof, it will be appreciated by those skilled in the art that additions, deletions, modifications, and substitutions not specifically described may be made without departing from the scope of the invention as defined in the appended claims.

## Claims

1. A system (10) for cooperative control of a surgical tool, comprising:
a surgical tool (16, 30) for use in vitreoretinal surgery and being adapted to be held by a robot (12) and a surgeon (17),
a tool holder (14) for receiving said surgical tool (16, 30);
a sensor (24) for detecting a force applied on a target area of interest of an eye by said surgical tool (16, 30);
a controller for limiting velocity of said robot (12) based upon the force detected so as to provide a haptic feedback;
a selector for automatically selecting an audio feedback based upon the force applied on the target area of interest of said eye by said surgical tool (16, 30), the audio feedback comprising a modulating playback of audio that varies in tempo based on the relative intensity of the force applied on said target area of interest of said eye by said surgical tool (16, 30); and
an audio device for providing the audio feedback together with the haptic feedback.

2. The system (10) of claim 1, wherein the audio feedback is a series of beeps.

3. The system (10) of claim 1, wherein the audio feedback is selected based upon whether the force applied on the target area of interest of said eye by said surgical tool (16, 30) falls within a predetermined range.

4. The system (10) of claim 3, wherein the audio feedback is silent until the force applied on the target area of interest of said eye by said surgical tool (16, 30) is more than 1 mN.

5. The system (10) of claim 3, wherein the audio feedback is a constant, slow tempo beeping when the force applied on the target area of interest of said eye by said surgical tool (16, 30) is in a predetermined range of between 1 mN and 3.5 mN.

6. The system (10) of claim 3, wherein the audio feedback is a constant, high tempo beeping when the force applied on the target area of interest of said eye by said surgical tool (16, 30) is in a predetermined range of between 3.5 mN to about 7 mN.

7. The system (10) of claim 6, wherein the high tempo beeping increases proportionally to the force applied on said target area of interest of said eye by said surgical tool (16, 30).

8. The system (10) of claim 1, wherein the surgical tool (16, 30) is an end effector in a surgical robot.

9. The system (10) of claim 1, wherein the sensor (24) is a fiber Bragg grating (FBG) sensor embedded in the surgical tool (30) for detecting the force between the surgical tool and the target area of interest of said eye.

## Patentansprüche

1. Ein System (10) zur kooperativen Steuerung eines chirurgischen Werkzeugs, das Folgendes beinhaltet:
ein chirurgisches Werkzeug (16, 30) zur Verwendung in vitreoretinaler Chirurgie, das angepasst ist, um von einem Roboter (12) und einem Chirurgen (17) gehalten zu werden,
einen Werkzeughalter (14) zum Aufnehmen des chirurgischen Werkzeugs (16, 30);
einen Sensor (24) zum Detektieren einer durch das chirurgische Werkzeug (16, 30) auf einen Zielbereich von Interesse eines Auges ausgeübten Kraft;
eine Steuereinheit zum Begrenzen der Geschwindigkeit des Roboters (12) basierend auf der detektierten Kraft, sodass haptisches Feedback bereitgestellt wird;
eine Auswahlvorrichtung zum automatischen Auswählen eines Audiofeedbacks basierend auf der durch das chirurgische Werkzeug (16, 30) auf den Zielbereich von Interesse des Auges ausgeübten Kraft, wobei das Audiofeedback das Modulieren einer Wiedergabe von Audio, die im Tempo variiert, basierend auf der relativen Intensität der durch das chirurgische Werkzeug (16, 30) auf den Zielbereich von Interesse des Auges ausgeübten Kraft beinhaltet; und
eine Audiovorrichtung zum Bereitstellen des Audiofeedbacks zusammen mit dem haptischen Feedback.

2. System (10) gemäß Anspruch 1, wobei das Audiofeedback eine Reihe von Pieptönen ist.

3. System (10) gemäß Anspruch 1, wobei das Audiofeedback basierend darauf ausgewählt ist, ob die durch das chirurgische Werkzeug (16, 30) auf den Zielbereich von Interesse des Auges ausgeübte Kraft in eine vorgegebene Spanne fällt.

4. System (10) gemäß Anspruch 3, wobei das Audiofeedback geräuschlos ist, bis die durch das chirurgische Werkzeug (16, 30) auf den Zielbereich von Interesse des Auges ausgeübte Kraft mehr als 1 mN beträgt.

5. System (10) gemäß Anspruch 3, wobei das Audiofeedback ein konstantes Piepen langsamen Tempos ist, wenn die durch das chirurgische Werkzeug (16, 30) auf den Zielbereich von Interesse des Auges ausgeübte Kraft in einer vorgegebenen Spanne von zwischen 1 mN und 3,5 mN liegt.

6. System (10) gemäß Anspruch 3, wobei das Audiofeedback ein konstantes Piepen hohen Tempos ist, wenn die durch das chirurgische Werkzeug (16, 30) auf den Zielbereich von Interesse des Auges ausgeübte Kraft in einer vorgegebenen Spanne von zwischen 3,5 mN und etwa 7 mN liegt.

7. System (10) gemäß Anspruch 6, wobei das Piepen hohen Tempos proportional zu der durch das chirurgische Werkzeug (16, 30) auf den Zielbereich von Interesse des Auges ausgeübten Kraft zunimmt.

8. System (10) gemäß Anspruch 1, wobei das chirurgische Werkzeug (16, 30) ein Endeffektor in einem chirurgischen Roboter ist.

9. System (10) gemäß Anspruch 1, wobei der Sensor (24) ein Faser-Bragg-Gitter(FBG)-Sensor ist, der zum Detektieren der Kraft zwischen dem chirurgischen Werkzeug und dem Zielbereich von Interesse des Auges in dem chirurgischen Werkzeug (30) eingebettet ist.

## Revendications

1. Un système (10) pour une commande coopérative d'un outil chirurgical, comprenant :
un instrument chirurgical (16, 30) pour une utilisation en chirurgie vitréo-rétinienne et étant conçu pour être tenu par un robot (12) et un chirurgien (17),
un support d'outil (14) pour recevoir ledit outil chirurgical (16, 30) ;
un capteur (24) pour détecter une force appliquée sur une zone cible d'intérêt d'un œil par ledit outil chirurgical (16, 30) ;
une unité de commande pour limiter la vitesse dudit robot (12) sur la base de la force détectée de façon à fournir un retour haptique ;
un sélectionneur pour automatiquement sélectionner un retour audio sur la base de la force appliquée sur la zone cible d'intérêt dudit œil par ledit outil chirurgical (16, 30), le retour audio comprenant la modulation d'une lecture audio dont le tempo varie sur la base de l'intensité relative de la force appliquée sur ladite zone cible d'intérêt dudit œil par ledit outil chirurgical (16, 30) ; et
un dispositif audio pour fournir le retour audio de pair avec le retour haptique.

2. Le système (10) de la revendication 1, dans lequel le retour audio est une série de bips.

3. Le système (10) de la revendication 1, dans lequel le retour audio est sélectionné sur la base du fait de savoir si la force appliquée sur la zone cible d'intérêt dudit œil par ledit outil chirurgical (16, 30) tombe dans les limites d'une gamme prédéterminée.

4. Le système (10) de la revendication 3, dans lequel le retour audio est silencieux jusqu'à ce que la force appliquée sur la zone cible d'intérêt dudit œil par ledit outil chirurgical (16, 30) est de plus de 1 mN.

5. Le système (10) de la revendication 3, dans lequel le retour audio est une émission de bips de tempo lent constant lorsque la force appliquée sur la zone cible d'intérêt dudit œil par ledit outil chirurgical (16, 30) se trouve dans une gamme prédéterminée comprise entre 1 mN et 3,5 mN.

6. Le système (10) de la revendication 3, dans lequel le retour audio est une émission de bips de tempo soutenu constant lorsque la force appliquée sur la zone cible d'intérêt dudit œil par ledit outil chirurgical (16, 30) se trouve dans une gamme prédéterminée comprise entre 3,5 mN et environ 7 mN.

7. Le système (10) de la revendication 6, dans lequel l'émission de bips de tempo soutenu augmente proportionnellement à la force appliquée sur ladite zone cible d'intérêt dudit œil par ledit outil chirurgical (16, 30).

8. Le système (10) de la revendication 1, dans lequel l'outil chirurgical (16, 30) est un effecteur terminal dans un robot chirurgical.

9. Le système (10) de la revendication 1, dans lequel le capteur (24) est un capteur de réseau de Bragg sur fibre (FBG) intégré à l'outil chirurgical (30) pour détecter la force entre l'outil chirurgical et la zone cible d'intérêt dudit œil.
